# EUROPÄISCHE PATENTSCHRIFT

(11) **EP 2 457 531 B1**
(45) Veröffentlichungstag und Bekanntmachung des Hinweises auf die Patenterteilung: **12.06.2019**
(21) Anmeldenummer: 11008988.5
(22) Anmeldetag: 11.11.2011
(51) Int. Cl.: A61B 17/88, B01F 11/00, B01F 13/00, B01F 15/02

(54) **Kartusche mit arretierbarem Förderkolben**
Cartridge with adjustable delivery pistons
Cartouche dotée de pistons de transport pouvant être bloqués

(30) Priorität: 25.11.2010 DE 102010052323
(43) Veröffentlichungstag der Anmeldung: 30.05.2012
(73) Patentinhaber: Heraeus Medical GmbH, 61273 Wehrheim (DE)
(72) Erfinder: Vogt, Sebastian, Dr., 99092 Erfurt (DE); Schnieber, Tim, 60439 Frankfurt (DE)
(74) Vertreter: Schultheiss & Sterzel Patentanwälte PartG mbB

(56) Entgegenhaltungen:
- WO-A1-01/85070
- WO-A1-94/26403
- WO-A1-94/26403
- WO-A1-2010/012114
- DE-B3-102009 031 178
- DE-B3-102009 031 178
- US-A1- 2005 128 867

## Beschreibung

Die Erfindung betrifft eine Kartusche zum Auspressen eines Kartuscheninhalts, insbesondere eines Zements, besonders bevorzugt eines medizinischen Zements, umfassend wenigstens einen durch eine Kartuschenwand begrenzten zylindrischen Hohlraum, zumindest einen im Hohlraum entlang der Zylinderachse des Hohlraums beweglich angeordneten Förderkolben, der an der Kartuschenwand anliegt, und wenigstens ein Rastmittel, mit dem der Förderkolben an der Kartuschenwand arretierbar ist.

Die Erfindung betrifft auch ein Kartuschensystem mit einer solchen Kartusche sowie ein Verfahren zur Verwendung einer solchen Kartusche. Knochenzemente aus Polymethylmethacrylat (PMMA) sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seit dem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Bei dem Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs bis dieser erstarrt. Polymethylmethacrylat-Knochenzemente können in geeigneten Mischbechern mit Hilfe von Spateln durch Vermischen des Zementpulvers mit der Monomerflüssigkeit gemischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerflüssigkeit in Vakuummischsystemen bevorzugt, da durch Mischen im Vakuum Lufteinschlüsse aus dem Zementteig weitgehend entfernt werden und damit eine optimale Zementqualität erreicht wird (Breusch S.J. et al.: Der Stand der Zementiertechnik in Deutschland. Z Orthop. 1999, 137: 101-07). Im Vakuum gemischte Knochenzemente haben eine deutlich verringerte Porosität und zeigen daher im ausgehärteten Zustand verbesserte mechanische Eigenschaften.

Es wurde eine Vielzahl von Vakuumzementiersystemen entwickelt, von denen exemplarisch die folgenden genannt seien: DE 36 40 279 A1, EP 1 020 167 A2, EP 1 016 452 A2, EP 1 005 901 A2, US 6,033,105 A, US 5,624,184 A, US 5,588,745 A, US 5,586,821 A, US 5,344,232 A, US 5,100,241 A, US 4,973,168 A, US 4,671,263 A, WO 99/67015 A1, WO 94/26403 A1.

Eine Weiterentwicklung stellen Zementiersysteme dar, in denen sowohl das Zementpulver als auch die Monomerflüssigkeit bereits in separaten Kompartimenten der Mischsysteme verpackt sind und erst unmittelbar vor der Zementapplikation im Zementiersystem miteinander vermischt werden (DE 10 2009 031 178 B3, US 5,997,544 A, EP 0 692 229 A1, US 6,709,149 B1).

Bei den meisten bisher bekannten Zementiersystemen besteht das Problem, dass während des Mischens, insbesondere beim Vakuummischen des Zements der im Zementbehälter axial verschiebbare Förderkolben fixiert sein muss und dass im Gegensatz dazu nach dem Mischen der Förderkolben axial beweglich sein muss, damit durch Druckeinwirkung einer Zementierpistole auf den Förderkolben das Herauspressen des Zementteigs infolge der axialen Bewegung des Förderkolbens erfolgen kann. Hierzu wurden unterschiedliche technischen Lösungen vorgeschlagen.

Die EP 0 861 117 A1 offenbart eine Zementiervorrichtung, bei der der Förderkolben durch einen beweglichen Splint gesichert ist, der nach dem Mischen des Zements gezogen werden kann, so dass dadurch der Kolben axial beweglich wird.

In der DE 43 02 230 A1 wird eine Zementiervorrichtung beschrieben, bei der der Förderkolben durch Stege fixiert wird, die in Aussparungen des Kartuschenbodens greifen. Durch Verdrehung des Förderkolbens gegenüber der Zementkartusche werden die Stege aus den Aussparungen gedreht und der Förderkolben wird entriegelt.

Eine völlig andere Fixierung des Förderkolbens wird in der WO 02/102287 A1 vorgeschlagen. Hierbei ist der Förderkolben mit dem Zementbehälter über eine brechbare Verbindung so verbunden, dass nur durch Brechen der Verbindung zwischen dem Förderkolben und dem Zementbehälter der Förderkolben beweglich wird.

Die US 2005/0128867 A1 offenbart eine Zementkartusche mit einem über eine Haltevorrichtung gegen die Kartusche arretierbaren Förderkolben, indem die Haltevorrichtung in Aussparungen an der Rückseite der Kartusche und in Aussparungen im Förderkolben greift, wobei die Haltevorrichtung mit Hilfe einer Scheibe einer Austragvorrichtung lösbar ist.

In der EP 2 008 707 A1 wird ein Verschluss für eine gattungsgemäße Kartusche beschrieben, bei der ein Förderkolben an der Außenseite und eine Kartuschenwand auf der Innenseite Aussparungen hat, in die innenseitige und außenseitige Rastmittel eines Rastrings greifen. Die Entriegelung des Förderkolbens erfolgt durch Anbringen einer Applikatorpistole und Überwinden der Rastung.

Nachteilig ist hieran, dass eine Applikatorpistole angesetzt werden muss, um den Förderkolben zu lösen. Zudem sind die Aussparungen Schwachstellen in der Kartuschenwand, auf die der Druck beim Auspressen des Kartuscheninhalts wirkt und an denen folglich die Kartuschen zerbrechen können.

Die Aufgabe der Erfindung besteht also darin, die Nachteile des Stands der Technik zu überwinden. Insbesondere soll eine einfache manuelle Entriegelung möglich sein. Auch soll der Aufbau einfacher und dadurch kostengünstiger werden. Gleichzeitig soll auch eine hohe Stabilität der Kartusche erzielt werden.

Es soll also eine Vorrichtung zum Austragen und wenn nötig auch zum Mischen von Knochenzement entwickelt werden, bei der die Verriegelung des Förderkolbens in einfachster Weise manuell gelöst werden kann, ohne dass lose Teile, wie Splinte freigesetzt werden. Weiterhin sollen zur Entriegelung keine Elemente einer äußeren Verpackung notwendig sein, wie zum Beispiel das Entriegeln durch Drehung der Zementkartusche gegen Stege eines Blisters oder das Ansetzen einer Applikatorpistole. Die Verriegelung, der Förderkolben und auch die Kartusche sollte weiterhin mit minimalem Aufwand zu fertigen sein.

Die Aufgabe der Erfindung wird dadurch gelöst, dass am Förderkolben zumindest eine Arretierungsvorrichtung angeordnet ist, die zumindest eines der Rastmittel umfasst und die bei arretiertem Förderkolben von außen zugänglich ist, jede Arretierungsvorrichtung ein Griffstück in Form einer Verlängerung umfasst und das Griffstück oder die Griffstücke zumindest bereichsweise aus dem Hohlraum herausragt oder herausragen, so dass die Arretierungsvorrichtung manuell entarretierbar ist.

Dadurch wird sichergestellt, dass die Entarretierung des Förderkolbens leicht möglich ist, wobei der Aufbau der Arretierungsvorrichtung gleichzeitig sehr einfach und daher kostengünstig zu realisieren ist.

Unter einem zylindrischen Körper beziehungsweise einer Kartusche mit zylindrischem Hohlraum sind nicht nur solche mit kreisförmigen Grundflächen zu verstehen, sondern ganz allgemein auch andere zylindrische Geometrien, beispielsweise mit ovalen oder eckigen Grundflächen. Der Innenraum der Kartusche kann neben dem zylindrischen Hohlraum erfindungsgemäß noch weitere nicht zylindrische Hohlräume umfassen.

Es ist erfindungsgemäß besonders vorteilhaft, wenn der Förderkolben entlang des gesamten Umfangs der Kartuschenwand an dieser anliegt, vorzugsweise dicht anliegt, besonders bevorzugt druckdicht anliegt.

Ferner kann vorgesehen sein, dass der Förderkolben mit der Arretierung arretiert ist.

Es kann auch vorgesehen sein, dass die Arretierungsvorrichtung oder die Arretierungsvorrichtungen alle Rastmittel umfassen.

Bei einer erfindungsgemäßen Austragsvorrichtung ist auch vorgesehen, dass jede Arretierungsvorrichtung ein Griffstück in Form einer Verlängerung umfasst und das Griffstück oder die Griffstücke zumindest bereichsweise aus dem Hohlraum herausragt oder herausragen, so dass die Arretierungsvorrichtung manuell entarretierbar ist.

Eine Weiterbildung der Erfindung sieht vor, dass die Arretierungsvorrichtung oder die Arretierungsvorrichtungen fest mit dem Förderkolben verbunden ist oder sind, vorzugsweise einteilig mit dem Förderkolben aufgebaut ist oder sind.

Eine besonders leicht manuell zu bedienende Ausführungsform ergibt sich erfindungsgemäß, wenn vorgesehen ist, dass zwei Arretierungsvorrichtungen am Förderkolben vorgesehen sind, die vorzugsweise gegenüberliegend zueinander angeordnet sind.

Eine besonders zweckmäßige, vorteilhafte Weiterentwicklung der Erfindung sieht vor, dass das Rastmittel oder die Rastmittel über den Rand der Kartuschenwand und/oder in eine Nut in der Kartuschenwand greifen.

Wenn eine Nut in der Kartuschenwand vorgesehen ist, kann dabei erfindungsgemäß vorgesehen sein, dass die Nut in einem Bereich der Kartuschenwand angeordnet ist, den der durch den an der Kartuschenwand anliegenden Teil des Förderkolbens beim Auspressen der Kartusche nicht überstreicht.

Ferner kann vorgesehen sein, dass der Förderkolben eine zylindrische Durchführung umfasst.

Dabei kann vorgesehen sein, dass in der Durchführung ein Mischstab angeordnet ist, der aus dem Hohlraum herausragt, mit dem ein Kartuscheninhalt im Hohlraum mischbar ist und der vorzugsweise eine Sollbruchstelle umfasst.

Dabei kann wiederum vorgesehen sein, dass an dem Mischstab ein Mischflügel im Inneren der Kartusche angeordnet ist.

Eine insbesondere im medizinischen Bereich geeignete Ausgestaltung der Erfindung ergibt sich, wenn der Förderkolben einen Vakuumanschluss umfasst, über den Gas aus dem Inneren der Kartusche evakuierbar ist.

Dabei kann vorgesehen sein, dass in der Gasdurchführung des Vakuumanschlusses vorzugsweise zumindest eine semipermeable Wand, insbesondere eine Porenscheibe angeordnet ist, die gasdurchlässig und pulverundurchlässig ist.

Zur einfachen Bedienbarkeit der Arretierungsvorrichtung kann vorgesehen sein, dass zumindest eine Arretierungsvorrichtung, vorzugsweise alle Arretierungsvorrichtungen, in Richtung der Zylinderachse des Hohlraums verschiebbar, kippbar und/oder biegbar ist oder sind und/oder vom Förderkolben abbrechbar ist oder sind, vorzugsweise manuell.

Eine noch leichter bedienbare Ausgestaltung der Erfindung ergibt sich, wenn die Rastelemente radial bezogen auf die Zylinderachse des Hohlraums zumindest bereichsweise über den Rand der Kartuschenwand herausragen, wenn der Förderkolben arretiert ist.

Ferner kann vorgesehen sein, dass der Hohlraum durch den Förderkolben einseitig abgeschlossen oder in einen Bereich im Inneren der Kartusche und einen von außen zugänglichen Bereich getrennt ist.

Zur Verbesserung der Dichtigkeit des Inneren der Kartusche kann vorgesehen sein, dass der Förderkolben zumindest einen Dichtungsring umfasst, der umlaufend an der Kartuschenwand anliegt.

Auch kann vorgesehen sein, dass der Förderkolben unterhalb des Rastmittels oder der Rastmittel jeweils in Verlängerung der Außenseiten des Rastmittels oder der Rastmittel zumindest einen Einschnitt im Förderkolbenmantel umfasst.

Ferner kann vorgesehen sein, dass die Kartusche an der dem Förderkolben in der arretierten Position gegenüberliegenden Seite eine Auslassöffnung, insbesondere ein Austragrohr zum Austragen des Kartuscheninhalts umfasst.

Dabei kann vorgesehen sein, dass im Bereich der Auslassöffnung der Kartusche ein Befestigungsmittel, insbesondere ein Gewinde, vorzugsweise ein Außengewinde angeordnet ist.

Mit Hilfe eines solchen Befestigungsmittels beziehungsweise Gewindes kann ein Austragsrohr an der Kartusche befestigt werden. Gleichzeitig kann die Kartusche auch an einem Träger befestigt werden, um ein Kartuschensystem zu bilden. Dabei kann die Kartusche mit einer Ampulle verbunden werden.

Ferner kann vorgesehen sein, dass die Kartusche zwei, drei oder mehrere Förderkolben in einem Hohlraum oder in verschiedenen, insbesondere parallel angeordneten Hohlräumen umfasst.

Die Aufgabe der Erfindung wird auch gelöst durch ein Kartuschensystem zur Herstellung eines Gemischs, vorzugsweise eines Zements, besonders bevorzugt eines medizinischen Zements umfassend zumindest eine solche Kartusche.

Dabei kann vorgesehen sein, dass das Kartuschensystem eine Ampulle mit einem flüssigen Inhalt, insbesondere eine Monomerampulle umfasst und die Kartusche ein Pulver, vorzugsweise ein Zementpulver beinhaltet.

Dabei kann vorgesehen sein, dass das Kartuschensystem einen Öffnungsmechanismus umfasst, mit dem die Ampulle zu öffnen ist, und der Inhalt der Ampulle über eine Leitung in die Kartusche leitbar ist, wobei die Leitung vorzugsweise an die Auslassöffnung der Kartusche angeschlossen ist.

Die Aufgabe der Erfindung wird schließlich auch gelöst durch ein Verfahren zum Austragen eines Kartuscheninhalts, bevorzugt eines Zements, besonders bevorzugt eines medizinischen Zements unter Verwendung einer solchen Kartusche, bevorzugt unter Verwendung eines solchen Kartuschensystems wobei der Förderkolben manuell entarretiert wird, bevor der Inhalt der Kartusche mit dem Förderkolben ausgepresst wird.

Dabei kann vorgesehen sein, dass der Kartuscheninhalt mit einem Mischstab durchmischt wird, der durch den Förderkolben geführt wird, bevor der Förderkolben entarretiert wird.

Es kann auch vorgesehen sein, dass die Kartusche ein Pulver, vorzugsweise ein Zementpulver beinhaltet und eine Flüssigkeit, vorzugsweise eine Monomerflüssigkeit in die Kartusche geleitet wird, bevor der Förderkolben entarretiert wird, insbesondere bevor der Kartuscheninhalt durchmischt wird.

Dabei ist es vorteilhaft, wenn zum Bereitstellen der Flüssigkeit eine Ampulle, vorzugsweise eine Monomerampulle geöffnet wird.

Eine Weiterentwicklung des erfindungsgemäßen Verfahrens ergibt sich, wenn vorgesehen ist, dass Gas aus dem Inneren der Kartusche durch den Förderkolben abgesaugt wird, wobei vorzugsweise die Flüssigkeit aus der Ampulle durch Einwirken eines Unterdrucks im Inneren der Kartusche in die Kartusche eingesaugt wird.

Auch kann insbesondere für medizinische Zwecke vorgesehen sein, dass das Innere der Kartusche und der Kartuscheninhalt mit einem Gas sterilisiert werden, vorzugsweise mit Ethylenoxid.

Unter einem manuellen Entarretieren des Förderkolbens kann auch die Verwendung einfacher, manuell bedienter Werkzeuge zu verstehen sein. Ebenso kann neben den Griffstücken auch eine andere zugängliche mechanische Vorrichtung vorgesehen sein, die eine manuell aufgebracht Kraft in die Entarretierung des Förderkolbens umsetzt.

Es wird auch eine Vorrichtung zum Mischen und Austragen von Knochenzement vorgeschlagen, die aus mindestens einem Zementbehälter, einem Mischorgan und einem Vakuumanschluss aufgebaut ist. Die Vorrichtung besitzt einen im Zementbehälter verschiebbaren Förderkolben, der mindestens ein Rastelement besitzt, das axial über die Ebene der Außenseite des Förderkolbens herausragt. Der Zementbehälter ist dabei eine zylindrische Kartusche, die mit einem Zementpulver gefüllt ist.

Die Erfindung wird auch realisiert durch eine Vorrichtung zum Mischen und Austragen von Knochenzement, die mindestens einen Zementbehälter (eine Kartusche), ein Mischorgan, einen Förderkolben mit wenigstens einem Dichtungselement und einen Vakuumanschluss umfasst, wobei mindestens ein Förderkolben in dem Zementbehälter verschiebbar angeordnet ist, an der zum Ende des Pulverbehälters zeigenden Öffnung mindestens ein in Richtung der Achse oder entgegengesetzt zur Achse des Förderkolbens biegbares oder verschiebbares Rastelement (Rastmittel) besitzt, wobei das Rastelement axial über die Ebene der Außenseite des Förderkolbens herausragt, das Rastelement mindestens eine Rastung besitzt, die in eine Nut des Zementbehälters oder über den Rand des Zementbehälters greift und die entgegengesetzt zur Achse des Förderkolbens gerichtete Außenfläche des Rastelements so groß ist, dass diese manuell in Richtung der Achse oder entgegengesetzt zur Achse des Förderkolbens gebogen oder geschoben werden kann.

Dabei kann vorgesehen sein, dass zwei Rastelelemente, bevorzugt gegenüberliegend zueinander angeordnet sind. Dadurch können mit einfachem Druck von Zeigefinger und Daumen die Rastelemente in Richtung Achse des Förderkolbens gedrückt oder geschoben werden und damit kann der Förderkolben entriegelt und axial im Zementbehälter (der Kartusche) verschoben werden.

Ferner kann vorgesehen sein, dass der Förderkolben unterhalb des Rastelements jeweils in Verlängerung der Außenseiten des Rastelements Einschnitte im Förderkolbenmantel besitzt. Dadurch kann das Rastelement leichter manuell mit Hilfe von Daumen und Zeigefinger in Richtung der Achse des Förderkolbens gedrückt oder geschoben werden. Es muss dabei nur beachtet werden, dass die Einschnitte oberhalb der Dichtungselemente des Förderkolbens enden.

Die Aufgabe wird auch gelöst durch die Verwendung einer solchen Kartusche, insbesondere unter Anwendung eines solchen Verfahrens, zum Auspressen von pastenförmigen Einkomponenten-Polymethylmethacrylat-Knochenzementen, von pastenförmigen Zweikomponenten-Polymethylmethacrylat-Knochenzementen, von pastenförmigen Dreikomponenten-Polymethylmethacrylat-Zementen, von dentalen Abformmassen, von anorganischen Knochenzementen und/oder von Polymethylmethacrylat-Knochenzementen, vorzugsweise durch Vermischen einer Pulverkomponente und einer flüssigen Monomerkomponente.

Der Erfindung liegt die überraschende Erkenntnis zugrunde, dass durch eine oder mehrere einfache Arretierungsvorrichtungen, die direkt am Förderkolben angeordnet ist oder sind, eine lösbare Befestigung des Förderkolbens an der Kartuschenwand erzielt werden kann. Der Aufbau ist dabei einfach und kostengünstig zu realisieren. Als Arretierungsvorrichtung reicht ein einfacher Haken am Förderkolben aus, der an das Ende der Kartuschenwand gehakt wird, an dem der Förderkolben im Ausgangszustand fest positioniert werden soll. Die Rastmittel, also beispielsweise die Füßchen des Hakens liegen dann außerhalb der Kartuschenwand und sind dadurch leicht zugänglich.

Besonders einfach bedienbar ist die Kartusche, wenn zusätzlich Griffstücke an den Arretierungsvorrichtungen angeordnet sind, mit denen sich die Rastmittel bequem lösen lassen. Dann muss die Gegenrastung auch nicht mehr am Ende der Kartuschenwand einhaken, sondern kann in eine Nut oder ein anderes Gegenrastmittel im Bereich des Förderkolbens greifen, da die manuelle Bedienbarkeit dadurch gegeben ist, dass die Griffstücke über die Kartuschenwand herausragen. Die Griffstücke oder das Griffstück sind zweckmäßigerweise ergonomisch geformt, um die Bedienung mit der Hand beziehungsweise mit Daumen und Zeigefinger zu ermöglichen. Ebenso kann aber auch ein Werkzeug oder Spezialwerkzeug zur Bedienung der Arretierungsvorrichtung vorgesehen sein.

Ein wesentlicher Vorteil der Erfindung ist auch darin zu sehen, dass die Kartuschenwand an den besonders belasteten Stellen nicht durch Ausnehmungen geschwächt wird. Eine Nut, die nicht im Inneren der Kartusche angeordnet ist, schwächt den Aufbau deshalb nicht, da dort kein Unterdruck beim Evakuieren des Inneren der Kartusche auftritt und auch bei Auspressen der Kartusche kein Druck durch den Förderkolben angreift. Solche Ausnehmungen stellen Sollbruchstellen für die Kartusche dar und müssen entweder aufwendig verstärkt werden oder die Kartuschenwand muss dicker, also mit mehr Material aufgebaut werden.

Erfindungsgemäß kann auch vorgesehen sein, dass die Rastung oberhalb oder auch unterhalb der Ebene der Außenseite des Kolbens angeordnet ist. Das bedeutet, dass die Rastung in Form einer Rastnase unterhalb der Ebene der Außenseite des Förderkolbens in eine umlaufende Nut greifen kann, die im Innenraum der Kartusche angeordnet ist. Bei der Anordnung der Rastung oberhalb der Ebene des Förderkolbens kann die Rastnase über den Rand der Kartusche ragen und dadurch eine axiale Bewegung des Förderkolbens im Hohlraum verhindern.

Im Folgenden werden Ausführungsbeispiele der Erfindung anhand von sieben schematisch dargestellten Figuren erläutert, ohne jedoch dabei die Erfindung zu beschränken. Dabei zeigt:
Figur 1: eine schematische Querschnittansicht eines erfindungsgemäßen Förderkolbens für eine erfindungsgemäße Kartusche;
Figur 2: eine schematische Querschnittansicht eines zweiten erfindungsgemäßen Förderkolbens mit einer Durchführung für eine erfindungsgemäße Kartusche;
Figur 3: eine schematische Seitenansicht eines erfindungsgemäßen Förderkolbens mit einer Durchführung mit zusammengedrücktem Rastelement;
Figur 4: eine schematische Querschnittansicht eines Bereichs einer erfindungsgemäßen Kartusche mit einem arretierten Förderkolben;
Figur 5: eine schematische Querschnittansicht der erfindungsgemäßen Kartusche mit entarretiertem und eingeschobenem Förderkolben;
Figur 6: eine schematische Querschnittansicht eines Bereichs einer zweiten erfindungsgemäßen Kartusche mit arretiertem Förderkolben; und
Figur 7: eine schematische Querschnittansicht eines erfindungsgemäßen Kartuschensystems mit einer erfindungsgemäßen Kartusche.

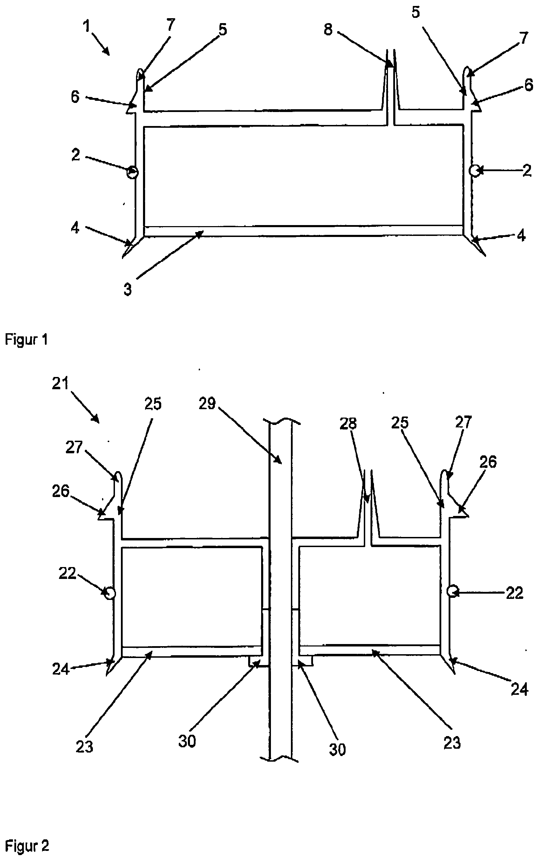

Figur 1 zeigt einen Förderkolben 1 für eine erfindungsgemäße Kartusche in schematischer Querschnittansicht. Der zylindrische Förderkolben 1 ist in seinem Inneren hohl und aus einem einfachen Kunststoff, beispielsweise durch ein Spritzgießverfahren hergestellt. Um den kreisrunden Umfang des Förderkolbens 1 ist eine elastische Dichtung 2 beispielsweise aus Gummi angeordnet. Die Unterseite des Förderkolbens 1 ist durch eine Porenscheibe 3 abgeschlossen. Am unteren Ende der Seitenwände des Förderkolbens 1 sind Abstreiflippen 4 angeordnet, die, wenn der Förderkolben 1 in eine erfindungsgemäße Kartusche eingesetzt ist, verhindern sollen, dass bei einer Bewegung des Förderkolbens 1 im Inneren der Kartusche (in Figur 1 nach unten) das zu befördernde Material im Inneren der Kartusche seitlich zwischen den Wänden des Förderkolbens 1 und den Innenwänden der Kartusche bis zur Dichtung 2 hindurch gedrückt wird. Die Abstreiflippen 4 sind dazu aus einem flexiblen, elastischen Material gefertigt. Die Elastizität der Abstreiflippen 4 lässt sich auch dadurch erreichen, indem ihre Materialstärke derart verringert wird, dass sich die gewünschte Elastizität auch bei Verwendung des selben Kunststoffs ergibt, aus dem auch der restliche Förderkolben 1 gefertigt ist.

Am oberen Ende der Seitenwände des Förderkolbens 1 sind zwei Arretierungsvorrichtungen 5 angeordnet, die jeweils ein Rastmittel 6 und ein Griffstück 7 umfassen. Wenn die beiden Arretierungsvorrichtungen 5 in Richtung der Zylinderachse zusammen gedrückt werden, indem sie beispielsweise an den Griffstücken 7 mit Zeigefinger und Daumen zusammengedrückt werden, so kippen die Arretierungsvorrichtungen in Richtung der Zylinderachse, wodurch die Rastmittel 6 ebenfalls geneigt werden. Wenn die Rastmittel 6 zuvor in eine Gegenrasterung (nicht gezeigt) an der Kartuschenwand oder über den Rand der Kartuschenwand gegriffen haben, so löst sich durch das zusammendrücken die Arretierung des Förderkolbens 1 mit der Kartuschenwand.

Auf der Oberseite des Förderkolbens 1 ist ein Vakuumanschluss 8 vorgesehen, durch den das Innere des Förderkolbens 1 evakuiert werden kann. Im eingebauten Zustand des Förderkolbens 1 lässt sich dann auch das unterhalb der Porenscheibe 3 angeordnete Innere der Kartusche evakuieren. Die Porenscheibe 3 ist gasdurchlässig, aber nicht für Pulver durchlässig. Dadurch ist es möglich, ein Gas durch die Porenscheibe 3 aus dem Inneren der Kartusche abzupumpen oder ein Gas in das Innere der Kartuschen einzuleiten, während ein im Inneren der Kartusche enthaltenes Pulver oder enthaltener Zement nicht durch die Porenscheibe 3 hindurch gelangen kann.

Für die Arretierungsfunktion des Förderkolbens 1 ist der Vakuumanschluss 8 und die Porenscheibe 3 überflüssig. Eine erfindungsgemäße Kartusche ergibt sich also auch dann, wenn statt der Porenscheibe 3 einfach ein einteiliger, massiver Förderkolben 1 mit einem Spritzgießverfahren hergestellt wird, ohne dass eine Öffnung, wie der Vakuumanschluss 8 vorgesehen ist. Der Vakuumanschluss 8 und die Porenscheibe 3 dienen ausschließlich dazu, einen Gasaustausch des Inneren der Kartusche zu ermöglichen, ohne dass ein in der Kartusche gelagertes Pulver austreten kann. Dies ist insbesondere bei einer medizinischen Anwendung vorteilhaft, da dann der Kartuscheninhalt durch Einspeisen eines sterilisierenden Gases, wie Ethylenoxid sterilisiert werden kann. Dies gilt auch für alle folgenden Ausführungsbeispiele.

Figur 2 zeigt eine schematische Querschnittansicht eines alternativen Förderkolbens 21 für eine erfindungsgemäße Kartusche. Auch dieser Förderkolben 21 ist zylindrisch aufgebaut. Um den Umfang des Förderkolbens 21 herum ist eine Gummidichtung 22 angeordnet. Der Förderkolben 21 ist bodenseitig durch eine semipermeable Porenscheibe 23 abgeschlossen. Im unteren Bereich des Förderkolbens 21 ist eine Abstreiflippe 24, die den Förderkolben 21 entlang des gesamten Umfangs umschließt, angeordnet. Im oberen Bereich des Förderkolbens 21 sind zwei Arretierungsvorrichtungen 25 angeordnet. Die Arretierungsvorrichtungen 25 umfassen jeweils ein Rastmittel 26 und ein Griffstück 27, mit dem die Arretierungsvorrichtungen 25 bedienbar sind.

Auf der Oberseite des Förderkolbens 21 ist ein Vakuumanschluss 28 angeordnet, über den der Förderkolben 21 und im eingebauten Zustand auch die Kartusche (nicht gezeigt) evakuierbar ist. Durch den Förderkolben 21 hindurch ist ein zylindrischer Mischstab 29 angeordnet, der durch eine Durchführung im Förderkolben 21 gelagert ist. In der Durchführung kann eine zweite Dichtung (nicht gezeigt) angeordnet sein. Im unteren Bereich der Durchführung ist eine Führungshülse 30 angeordnet, die den Mischstab 29 führt, die semipermeable Porenscheibe 23 hält und den Förderkolben 21 gegen den Mischstab 29 abdichtet.

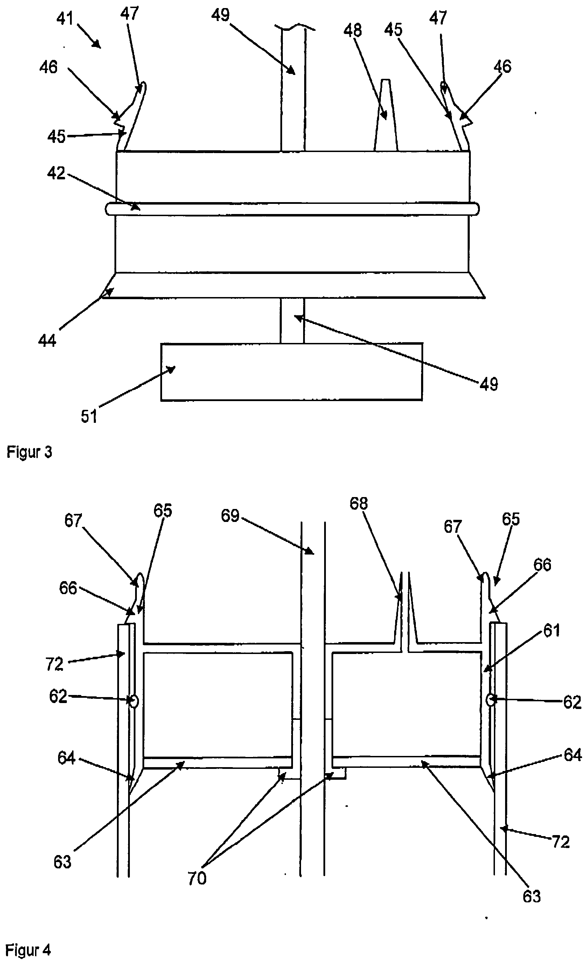

Figur 3 zeigt eine schematische Seitenansicht eines Förderkolbens 41 für eine erfindungsgemäße Kartusche. Eine Gummidichtung 42 umgibt den Förderkolben 41 entlang des gesamten Umfangs. Am unteren Rand des Förderkolbens 41 ist eine flexible Abstreiflippe 44 angeordnet, die den Förderkolben 41 ebenfalls vollumfänglich umschließt. Am oberen Rand des Förderkolbens 41 sind Arretierungsvorrichtungen 45 vorgesehen, mit der der Förderkolben 41 an einer Kartuschenwand (nicht gezeigt) der Kartusche befestigt werden kann. Die Arretierungsvorrichtungen 45 umfassen ein Rastmittel 46 und ein Griffstück 47. Durch eine Krafteinwirkung an den Griffstücken 47 sind die Arretierungsvorrichtungen 45 in Richtung des Zentrums des Förderkolbens 41 geneigt. Hierdurch sind auch die Rastmittel 46 in Richtung des Zentrums des Förderkolbens 41 bewegt worden, wodurch die Arretierung der Rastmittel 46 mit der Kartuschenwand aufgehoben ist. Der Förderkolben 41 ist dann frei im Inneren der Kartusche beweglich.

Auf der Oberseite des Förderkolbens 41 ist ein Vakuumanschluss 48 angeordnet. Durch das Zentrum des Förderkolbens 41 hindurch ist eine Stange eines Mischstabs 49 beweglich (drehbar und verschiebbar) im Förderkolben 41 angeordnet. An der Unterseite des Mischstabs 49, der im eingebauten Zustand des Förderkolbens 41 im Inneren der Kartusche angeordnet ist, ist ein Mischflügel 51 vorgesehen, mit dem der Kartuscheninhalt durchmischt werden kann.

Figur 4 zeigt eine schematische Querschnittansicht eines Teils einer erfindungsgemäßen Kartusche mit einem arretierten Förderkolben 61. Zur Abdichtung des Inneren der Kartusche (in Figur 4 unten) ist am Förderkolben 61 eine Dichtung 62 vorgesehen. Die dem Inneren der Kartusche zugewandte Unterseite des Förderkolbens 61 wird durch eine gasdurchlässige Porenscheibe 63 gebildet. Der äußere Rand des Förderkolbens 61 wird an der Unterseite durch eine umlaufende Abstreiflippe 64 begrenzt, die verhindern soll, dass Material aus dem Inneren der Kartusche am Förderkolben 61 vorbei nach außen gedrückt werden kann. An der Oberseite des Förderkolbens 61 sind außen zwei Arretierungsvorrichtung 65 angeordnet, die jeweils ein Rastmittel 66 und ein Griffstück 67 umfassen. Auf der Oberseite des Förderkolbens 61 ist zudem ein Vakuumanschluss 68 angeordnet, auf den ein Schlauch aufgesteckt werden kann. Durch das Zentrum des Förderkolbens 61 führt ein Mischstab 69, der durch eine Führungshülse 70 abgedichtet und positioniert wird. Der Mischstab 69 ist drehbar im Förderkolben 61 gelagert.

Der Förderkolben 61 steckt im oberen Teil einer Kartusche mit einem zylindrischen Hohlraum, der durch eine Kartuschenwand 72 begrenzt ist. Die unteren Flächen der Rastmittel 66 liegen auf der Oberkante der Kartuschenwand 72 auf und verhindern so, dass der Förderkolben 61 ins Innere der Kartusche (in Figur 4 nach unten) verschiebbar ist. Figur 4 zeigt also die arretierte Position des Förderkolbens 61 in der Kartusche.

Auch wenn über den Vakuumanschluss 68 das Innere der Kartusche evakuiert wird, kann der arretierte Förderkolben 61 nicht ins Innere der Kartuschen gezogen werden. Die Arretierung verhindert auch eine Bewegung des Förderkolbens 61, wenn der Mischstab 69 bedient wird.

Zum Lösen des Förderkolbens 61 müssen die Griffstücke 67 der Arretierungsvorrichtung 65 lediglich manuell oder mit Hilfe eines einfachen Werkzeugs in Richtung des Mischstabs 69 gedrückt werden. Wenn die Kartusche für den einmaligen Gebrauch gedacht ist, können die Arretierungsvorrichtungen 65 auch reversibel oder irreversibel verformt oder sogar abgebrochen werden, um den Förderkolben 61 zu Entarretieren.

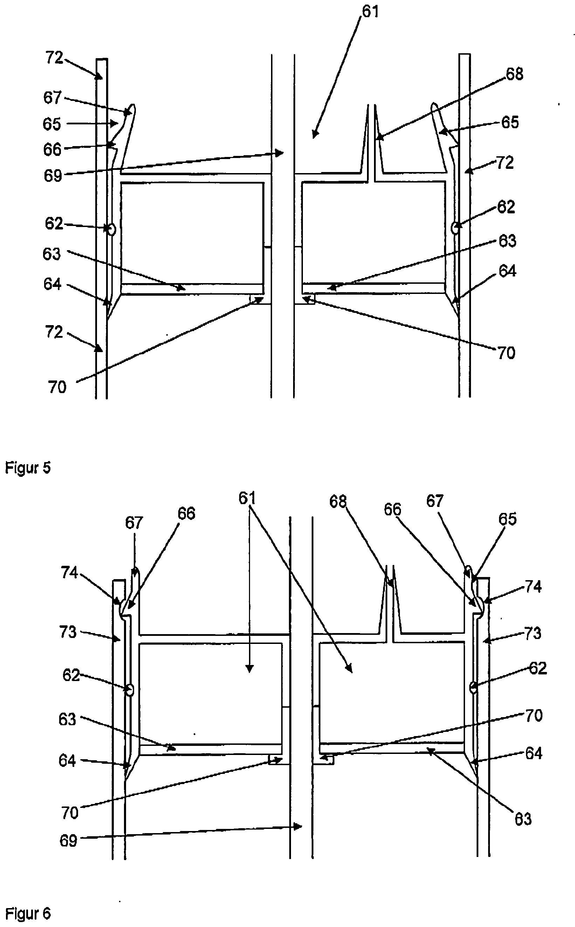

Figur 5 zeigt in einer schematischen Querschnittansicht den gleichen Aufbau nach Figur 4 mit entarretiertem Förderkolben 61. Die Arretierungsvorrichtungen 65 sind nach innen gebogen, so dass die Rastmittel 66 nicht mehr auf die Oberkante der Kartuschenwand 72 greifen. Aufgrund eines auf die Oberseite des Förderkolbens 61 wirkenden Drucks, der durch eine Evakuierung des Inneren der Kartusche und/oder durch eine mechanisch wirkende Kraft von oben auf den Förderkolben 61 verursacht werden kann, ist der Förderkolben 61 ins Innere der Kartusche (in Figur 5 nach unten) verschoben worden. Die Abstreiflippen 64 verhindern dabei, dass Material, das von dem sich bewegenden Förderkolben 61 nach unten gepresst wird, zwischen die Außenwand des Förderkolbens 61 und die Kartuschenwand 72 gelangen kann.

Figur 6 zeigt einen alternativen schematischen Aufbau einer erfindungsgemäßen Kartusche in Querschnittansicht mit dem gleichen Förderkolben 61, wie nach den Figuren 4 und 5. Im Unterschied zu den Figuren 4 und 5 sind in der Kartuschenwand 73 nach Figur 6 Nuten 74 vorgesehen, die als Gegenrastmittel für die Rastmittel 66 der Arretierungsvorrichtungen 65 dienen. Die Griffstücke 67 der Arretierungsvorrichtungen 65 sind lang genug, um über den oberen Rand der Kartuschenwand 73 und damit aus dem durch die Kartuschenwand 73 begrenzten zylindrischen Hohlraum herauszuragen. Damit bleiben die Griffstücke 67 und damit die Arretierungsvorrichtung 65 manuell zugänglich.

Durch Zusammendrücken der Griffstücke 67 rutschen die Rastmittel 66 aus den Nuten 74 und geben so den Förderkolben 61 frei. Der Förderkolben 61 ist anschließend im zylindrischen Hohlraum beweglich. Die Gummidichtung 62 liegt entlang des gesamten Umfangs des Förderkolbens 61 gasdicht und druckdicht an der Kartuschenwand 73 an. Dadurch kann im Inneren der Kartusche (in Figur 6 unten) ein Unterdruck erzeugt werden, ohne dass Gas zwischen dem Förderkolben und der Kartuschenwand ins Innere der Kartusche eindringen kann.

Auf der dem Förderkolben 61 gegenüberliegenden Seite der Kartusche (bezogen auf Figur 6 unten außerhalb des Bilds) ist eine Auslassöffnung (nicht gezeigt) angeordnet, durch die der Kartuscheninhalt mit Hilfe des Förderkolbens 61 aus der Kartusche herausgepresst werden kann, nachdem der Förderkolben 61 entarretiert wurde.

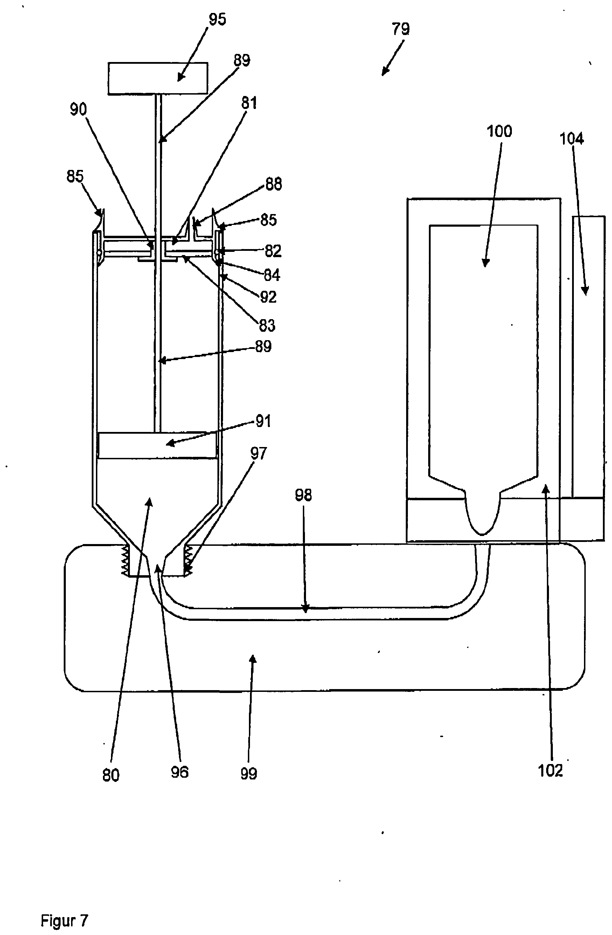

Figur 7 zeigt in schematischer Querschnittansicht den Aufbau eines erfindungsgemäßen Kartuschensystems 79 mit einer erfindungsgemäßen Kartusche 80. In der Kartusche 80 ist ein arretierter Förderkolben 81 angeordnet, der im entarretierten Zustand in Längsrichtung der Kartusche 80 im Inneren der Kartusche 80 beweglich angeordnet ist. Der Förderkolben 81 umfasst eine Dichtung 82 und eine Abstreiflippe 84, die den Förderkolben 81 vollumfänglich umschließen. Zwei Arretierungsvorrichtungen 85 sind auf der Oberseite des Förderkolbens 81 am Förderkolben 81 angeordnet. Auf der Oberseite des Förderkolbens 81 ist ein Vakuumanschluss 88 vorgesehen, über den das Innere der Kartusche 80 evakuierbar ist.

Ein Mischstab 89 erstreckt sich durch eine Führungshülse 90 durch den Förderkolben 81. Im Inneren der Kartusche 80 ist ein Mischflügel 91 am Mischstab 89 angeordnet. Der Mischstab 89 ist drehbar im Förderkolben 81 gelagert, so dass der Mischflügel 91 in der Kartusche 80 drehbar ist. Das Innere der Kartusche 80 ist durch eine zylindrische Kartuschenwand 92 begrenzt, so dass im Inneren der Kartusche 80 ein zylindrischer Hohlraum gebildet wird, der, bis auf den unteren trichterförmigen Teil des Innenraums (in Figur 7 im unteren Bereich des Kartuscheninnenraums), das Innere der Kartusche 80 bildet.

Am oberen Ende des Mischstabs 89 ist ein Griff 95 angeordnet, mit dem der Mischstab 89 manuell oder auch über einen Motor bedienbar ist. Auf der dem arretierten Förderkolben 81 gegenüberliegenden Seite der Kartusche 80 mündet der Kartuscheninnenraum in eine Auslassöffnung 96, durch die ein in der Kartusche 80 enthaltenes Material mit dem entarretierten Förderkolben 81 auspressbar ist. Die Kartusche 80 hat im Bereich der Auslassöffnung 96 ein Außengewinde 97, um die Kartusche 80 mit einer Leitung 98 eines Trägers 99 zu verbinden. Dazu wird die Kartusche 80 in ein Innengewinde des Trägers 99 geschraubt.

Auf der anderen Seite des Trägers 99 ist eine Ampulle 100 mit einer Monomerflüssigkeit in einem Behälter 102 angeordnet. Die Ampulle 100 kann über einen Öffnungsmechanismus 104 geöffnet werden, der den Kopf der Ampulle 100 abschert.

Die Kartusche 80 ist zu ca. 2/3 mit einem Knochenzementpulver gefüllt. Zur Sterilisierung des Inhalts wird das Innere der Kartusche 80 zunächst über den Vakuumanschluss 88 evakuiert. Anschließend wird ein sterilisierendes Gas, wie beispielsweise Ethylenoxid in die Kartusche 80 geleitet. Nach einer Zeit, die zum Sterilisieren des Inhalts der Kartusche 80 ausreicht, wird das Ethylenoxid wieder abgepumpt.

Mit Hilfe des Öffnungsmechanismus 104 wird die Ampulle 100 geöffnet und die Monomerflüssigkeit fließt in die Leitung 98. Aufgrund des Unterdrucks im Inneren der Kartusche 80 wird die Monomerflüssigkeit in die Kartusche 80 gezogen und mischt sich dort mit dem Zementpulver. Mit Hilfe des Mischstabs 89 und des Mischflügels 91 können die Monomerflüssigkeit und das Zementpulver durchmischt werden. Aufgrund des Vakuums entstehen in dem entstehenden Zementgemisch keine unerwünschten Lufteinschlüsse. Nach der Durchmischung wird der Förderkolben 81 an den Arretierungsvorrichtungen 85 manuell entarretiert. Da im Inneren der Kartusche 80 ein geringerer Druck als in der Umgebung wirkt, wird der Förderkolben 81 ins Innere der Kartusche 80 gezogen. Die Kartusche 80 wird an der Vorderseite geöffnet, beispielsweise in dem ein Ventil (nicht gezeigt) bedient wird, oder die Kartusche 80 aus dem Träger 99 herausgeschraubt wird. Durch Eindrücken des Förderkolbens 81 wird dann das Zementgemisch aus dem Inneren des Förderkolbens 81 durch die Auslassöffnung 96 herausgepresst.

Auf das Gewinde 97 kann ein Austragsrohr geschraubt werden, so dass der Knochenzement leicht an der gewünschten Stelle appliziert werden kann.

### Bezugszeichenliste

- 1, 21, 41, 61, 81: Förderkolben
- 2, 22, 42, 62, 82: Dichtung
- 3, 23, 63, 83: Porenscheibe
- 4, 24, 44, 64, 84: Abstreiflippe
- 5, 25, 45, 65, 85: Arretierungsvorrichtung
- 6, 26, 46, 66: Rastmittel
- 7, 27, 47, 67: Griffstück
- 8, 28, 48, 68, 88: Vakuumanschluss
- 29, 49, 69, 89: Mischstab
- 30, 70, 90: Führungshülse
- 51, 91: Mischflügel
- 72, 73, 92: Kartuschenwand
- 74: Nut
- 79: Kartuschensystem
- 80: Kartusche
- 95: Griff
- 96: Auslassöffnung
- 97: Gewinde
- 98: Leitung
- 99: Träger
- 100: Ampulle
- 102: Behälter
- 104: Öffnungsmechanismus

## Patentansprüche

1. Kartusche (80) zum Auspressen eines Kartuscheninhalts, insbesondere eines Zements, besonders bevorzugt eines medizinischen Zements, umfassend wenigstens einen durch eine Kartuschenwand (72, 73, 92) begrenzten zylindrischen Hohlraum, zumindest einen im Hohlraum entlang der Zylinderachse des Hohlraums beweglich angeordneten Förderkolben (1, 21, 41, 61, 81), der an der Kartuschenwand (72, 73, 92) anliegt, und wenigstens ein Rastmittel (6, 26,46, 66), mit dem der Förderkolben (1, 21, 41, 61, 81) an der Kartuschenwand (72,73, 92) arretierbar ist, wobei am Förderkolben (1, 21,41,61, 81) zumindest eine Arretierungsvorrichtung (5, 25, 45, 65,85) angeordnet ist, die zumindest eines der Rastmittel (6, 26, 46, 66) umfasst und die bei arretiertem Förderkolben (1, 21,41,61, 81) von außen zugänglich ist, **dadurch gekennzeichnet, dass** jede Arretierungsvorrichtung (5, 25,45,65,85) ein Griffstück (7, 27, 47, 67) in Form einer Verlängerung umfasst und das Griffstück (7, 27, 47, 67) oder die Griffstücke (7, 27, 47, 67) zumindest bereichsweise aus dem Hohlraum herausragt oder herausragen, so dass die Arretierungsvorrichtung (6, 25, 45,65, 85) manuell entarretierbar ist.

2. Kartusche (80) nach Anspruch 1, **dadurch gekennzeichnet, dass**
die Arretierungsvorrichtung (5, 25, 45, 65, 85) oder die Arretierungsvorrichtungen (5,25, 45,65, 85) fest mit dem Förderkolben (1, 21, 41, 61, 81) verbunden ist oder sind, vorzugsweise einteilig mit dem Förderkolben (1, 21, 41, 61, 81) aufgebaut ist oder sind.

3. Kartusche (80) nach einem der vorangehenden Ansprüche, **dadurch gekennzeichnet, dass** zwei Arretierungsvorrichtungen (5, 25, 45, 65, 85) am Förderkolben (1, 21, 41, 61, 81) vorgesehen sind, die vorzugsweise gegenüberliegend zueinander angeordnet sind.

4. Kartusche (80) nach einem der vorangehenden Ansprüche, **dadurch gekennzeichnet, dass** das Rastmittel (6, 26, 46, 66) oder die Rastmittel (6, 26, 46, 66) über den Rand der Kartuschenwand (72, 73, 92) greift oder greifen.

5. Kartusche (80) nach einem der vorangehenden Ansprüche, **dadurch gekennzeichnet, dass** das Rastmittel (6, 26, 46, 66) oder die Rastmittel (6, 26, 46, 66) in eine Nut (74) in der Kartuschenwand (72, 73, 92) greift oder greifen, wobei die Nut (74) in einem Bereich der Kartuschenwand (72, 73, 92) angeordnet ist, den der durch den an der Kartuschenwand (72, 73, 92) anliegenden Teil des Förderkolbens (1, 21, 41, 61, 81) beim Auspressen der Kartusche (80) nicht überstreicht.

6. Kartusche (80) nach einem der vorangehenden Ansprüche, **dadurch gekennzeichnet, dass** der Förderkolben (1, 21, 41, 61, 81) eine zylindrische Durchführung umfasst.

7. Kartusche (80) nach Anspruch 6, **dadurch gekennzeichnet, dass**
In der Durchführung ein Mischstab (29, 49, 69, 89) angeordnet ist, der aus dem Hohlraum herausragt, mit dem ein Kartuscheninhalt im Hohlraum mischbar ist und der vorzugsweise eine Sollbruchstelle umfasst

8. Kartusche (80) nach Anspruch 7, **dadurch gekennzeichnet, dass**
an dem Mischstab (29, 49, 69, 89) ein Mischflügel (51, 91) im Inneren der Kartusche (80) angeordnet ist.

9. Kartusche (80) nach einem der vorangehenden Ansprüche, **dadurch gekennzeichnet, dass** der Förderkolben (1, 21, 41, 61, 81) einen Vakuumanschluss (8, 28, 48, 68, 88) umfasst, über den Gas aus dem Inneren der Kartusche (80) evakuierbar ist, wobei in der Gasdurchführung des Vakuumanschlusses (8, 28, 48, 68, 88) vorzugsweise zumindest eine semipermeable Wand (3, 23, 63, 83), insbesondere eine Porenscheibe (3, 23, 63, 83) angeordnet ist, die gasdurchlässig und pulverundurchlässig ist.

10. Kartusche (80) nach einem der vorangehenden Ansprüche, **dadurch gekennzeichnet, dass**
zumindest eine Arretierungsvorrichtung (5, 25, 45, 65, 85), vorzugsweise alle Arretierungsvorrichtungen (5, 25, 45, 65, 85), in Richtung der Zylinderachse des Hohlraums verschiebbar, kippbar und/oder biegbar ist oder sind und/oder vom Förderkolben (1, 21, 41, 61, 81) abbrechbar ist oder sind, vorzugsweise manuell.

11. Kartusche (80) nach einem der vorangehenden Ansprüche, **dadurch gekennzeichnet, dass**
die Rastmittel radial bezogen auf die Zylinderachse des Hohlraums zumindest bereichsweise über den Rand der Kartuschenwand (72, 73, 92) herausragen, wenn der Förderkolben (1, 21, 41, 61, 81) arretiert ist.

12. Kartusche (80) nach einem der vorangehenden Ansprüche, **dadurch gekennzeichnet, dass**
der Hohlraum durch den Förderkolben (1, 21, 41, 61, 81) einseitig abgeschlossen oder
in einen Bereich im Inneren der Kartusche (80) und einen von außen zugänglichen Bereich getrennt ist.

13. Kartusche (80) nach einem der vorangehenden Ansprüche, **dadurch gekennzeichnet, dass**
der Förderkolben (1, 21, 41, 61, 81) zumindest einen Dichtungsring (2, 22, 42, 62, 82) umfasst, der umlaufend an der Kartuschenwand (72, 73, 92) anliegt.

14. Kartusche (80) nach einem der vorangehenden Ansprüche, **dadurch gekennzeichnet, dass**
der Förderkolben (1, 21, 41, 61, 81) unterhalb des Rastmittels (6, 26, 46, 66) oder der Rastmittel (6, 26, 46, 66) jeweils in Verlängerung der Außenseiten des Rastmittels (6, 26, 46, 66) oder der Rastmittel (6, 26, 46, 66) zumindest einen Einschnitt im Förderkolbenmantel umfasst.

15. Kartusche (80) nach einem der vorangehenden Ansprüche, **dadurch gekennzeichnet, dass**
die Kartusche (80) an der dem Förderkolben (1, 21, 41, 61, 81) in der arretierten Position gegenüberliegenden Seite eine Auslassöffnung (96), insbesondere ein Austragrohr zum Austragen des Kartuscheninhalts umfasst.

16. Kartusche (80) nach Anspruch 15, **dadurch gekennzeichnet, dass**
im Bereich der Auslassöffnung (96) der Kartusche (80) ein Befestigungsmittel (97), insbesondere ein Gewinde (97), vorzugsweise ein Außengewinde (97) angeordnet ist.

17. Kartuschensystem (79) zur Herstellung eines Gemischs, vorzugsweise eines Zements, besonders bevorzugt eines medizinischen Zements umfassend zumindest eine Kartusche (80) nach einem der vorangehenden Ansprüche.

18. Kartuschensystem (79) nach Anspruch 17, **dadurch gekennzeichnet, dass**
das Kartuschensystem (79) eine Ampulle (100) mit einem flüssigen Inhalt, insbesondere eine Monomerampulle (100) umfasst und die Kartusche (80) ein Pulver, vorzugsweise ein Zementpulver beinhaltet.

19. Kartuschensystem (79) nach Anspruch 18, **dadurch gekennzeichnet, dass**
das Kartuschensystem (79) einen Öffnungsmechanismus (104) umfasst, mit dem die Ampulle (100) zu öffnen ist, und der Inhalt der Ampulle (100) über eine Leitung (98) in die Kartusche (80) leitbar ist, wobei die Leitung (98) vorzugsweise an die Auslassöffnung (96) der Kartusche (80) angeschlossen ist.

20. Verfahren zur Verwendung einer Kartusche nach einem der Ansprüche 1 bis 16, bevorzugt unter Verwendung eines Kartuschensystems nach einem der Ansprüche 17 bis 19, **dadurch gekennzeichnet, dass** der Förderkolben manuell entarretiert wird.

21. Verfahren nach Anspruch 20, **dadurch gekennzeichnet, dass**
der Kartuscheninhalt mit einem Mischstab durchmischt wird, der durch den Förderkolben geführt wird, bevor der Förderkolben entarretiert wird.

22. Verfahren nach einem der Ansprüche 20 oder 21, **dadurch gekennzeichnet, dass** die Kartusche ein Pulver, vorzugsweise ein Zementpulver beinhaltet und eine Flüssigkeit, vorzugsweise eine Monomerflüssigkeit in die Kartusche geleitet wird, bevor der Förderkolben entarretiert wird, insbesondere bevor der Kartuscheninhalt durchmischt wird.

23. Verfahren nach Anspruch 22, **dadurch gekennzeichnet, dass**
zum Bereitstellen der Flüssigkeit eine Ampulle, vorzugsweise eine Monomerampulle geöffnet wird.

24. Verfahren nach einem der Ansprüche 20 bis 23, **dadurch gekennzeichnet, dass** Gas aus dem Inneren der Kartusche durch den Förderkolben abgesaugt wird, wobei vorzugsweise die Flüssigkeit aus der Ampulle durch Einwirken eines Unterdrucks im Inneren der Kartusche in die Kartusche eingesaugt wird.

25. Verfahren nach einem der Ansprüche 20 bis 23, **dadurch gekennzeichnet, dass** das Innere der Kartusche und der Kartuscheninhalt mit einem Gas sterilisiert werden, vorzugsweise mit Ethylenoxid.

## Claims

1. Cartridge (80) for pressing out a cartridge content, in particular a cement, in particular preferably a medical cement, comprising at least one cylindrical cavity bounded by a cartridge wall (72, 73, 92), at least one conveying piston (1, 21, 41, 61, 81) arranged movably in the cavity along the cylinder axis of the cavity and bearing against the cartridge wall (72, 73, 92), and at least one latching means (6, 26, 46, 66) with which the conveying piston (1, 21, 41, 61, 81) can be locked on the cartridge wall (72, 73, 92), at least one locking device (5, 25, 45, 65, 85), which comprises at least one of the latching means (6, 26, 46, 66) and which is accessible from the outside when the conveying piston (1, 21, 41, 61, 81) is locked, **characterized in that** each locking device (5, 25, 45, 65, 85) comprises a grip piece (7, 27, 47, 67) in the form of an extension and the grip piece (7, 27, 47, 67) or the grip pieces (7, 27, 47, 67) protrudes or protrude at least regionally from the cavity so that the locking device (5, 25, 45, 65, 85) can be unlocked manually.

2. A cartridge (80) according to claim 1, **characterized in that**
the locking device (5, 25, 45, 65, 85) is or the locking devices (5, 25, 45, 65, 85) are fixedly connected to the conveying piston (1, 21, 41, 61, 81), preferably constructed in one piece with the conveying piston (1, 21, 41, 61, 81).

3. A cartridge (80) according to one of the preceding claims, **characterized in that**
two locking devices (5, 25, 45, 65, 85) are provided on the conveying piston (1, 21, 41, 61, 81), which are preferably arranged opposite one another.

4. A cartridge (80) according to one of the preceding claims, **characterized in that**
the latching means (6, 26, 46, 66) grasps or latching means (6, 26, 46, 66) grasp over an edge of a cartridge wall (72, 73, 92).

5. A cartridge (80) according to one of the preceding claims, **characterized in that**
the latching means (6, 26, 46, 66) engages or engage in a groove (74) in the cartridge wall (72, 73, 92), the groove (74) being arranged in a region of the cartridge wall (72, 73, 92) which is not swept by the part of the conveying piston (1, 21, 41, 61, 81) which bears against the cartridge wall (72, 73, 92) when the cartridge (80) is pressed out.

6. A cartridge (80) according to one of the preceding claims, **characterized in that**
the conveying piston (1, 21, 41, 61, 81) comprises a cylindrical bushing.

7. A cartridge (80) according to claim 6, **characterized in that**
a mixing rod (29, 49, 69, 89) is arranged in the bushing, which projects from the cavity, with which a cartridge content in the cavity can be mixed and which preferably comprises a predetermined breaking point.

8. A cartridge (80) according to claim 7, **characterized in that**
a mixing blade (51, 91) is arranged on the mixing rod (29, 49, 69, 89) inside the cartridge (80).

9. A cartridge (80) according to one of the preceding claims, **characterized in that**
the conveying piston (1, 21, 41, 61, 81) comprises a vacuum connection (8, 28, 48, 68, 88) via which gas is evacuable from the interior of the cartridge (80), at least one semipermeable wall (3, 23, 63, 83), in particular a pore disc (3, 23, 63, 83), which is gas-permeable and powder-impermeable preferably being arranged in the gas passage of the vacuum connection (8, 28, 48, 68, 88).

10. A cartridge (80) according to one of the preceding claims, **characterized in that**
at least one locking device (5, 25, 45, 65, 85), preferably all locking devices (5, 25, 45, 65, 85), is or are displaceable, tiltable and/or flexible in the direction of the cylinder axis of the cavity and/or is or are breakable from the conveying piston (1, 21, 41, 61, 81), preferably manually.

11. A cartridge (80) according to one of the preceding claims, **characterized in that**
the latching means project radially relative to the cylinder axis of the cavity at least regionally beyond the edge of the cartridge wall (72, 73, 92) when the conveying piston (1, 21, 41, 61, 81) is locked.

12. A cartridge (80) according to one of the preceding claims, **characterized in that**
the cavity is closed at one end by the conveying piston (1, 21, 41, 61, 81) or is separated into a section inside the cartridge (80) and a section accessible from the outside.

13. A cartridge (80) according to one of the preceding claims, **characterized in that**
the conveying piston (1, 21, 41, 61, 81) comprises at least one sealing ring (2, 22, 42, 62, 82) which bears circumferentially against the cartridge wall (72, 73, 92).

14. A cartridge (80) according to one of the preceding claims, **characterized in that**
the conveying piston (1, 21, 41, 61, 81) comprises at least one incision in the conveying pistons cylinder barrel below the latching means (6, 26, 46, 66) in each case in extension of the outer sides of the latching means (6, 26, 46, 66).

15. A cartridge (80) according to one of the preceding claims, **characterized in that**
the cartridge (80) comprises, on the side opposite the conveying piston (1, 21, 41, 61, 81) in the locked position, an outlet opening (96), in particular a discharge pipe, for discharging the cartridge contents.

16. A cartridge (80) according to claim 15, **characterized in that**
a fastening means (97), in particular a thread (97), preferably an external thread (97), is arranged in the region of the outlet opening (96) of the cartridge (80).

17. A cartridge system (79) for producing a mixture, preferably a cement, particularly preferably a medical cement, comprising at least one cartridge (80) according to one of the preceding claims.

18. A cartridge system (79) according to claim 17, **characterized in that**
the cartridge system (79) comprises an ampoule (100) with a liquid content, in particular a monomer ampoule (100), and the cartridge (80) contains a powder, preferably a cement powder.

19. A cartridge system (79) according to claim 18, **characterized in that**
the cartridge system (79) comprises an opening mechanism (104) with which the ampoule (100) is openable, and the contents of the ampoule (100) can be conducted into the cartridge (80) via a line (98), the line (98) preferably being connected to the outlet opening (96) of the cartridge (80).

20. Method using a cartridge according to one of claims 1 to 16, preferably using a cartridge system according to one of claims 17 to 19, **characterized in that**
the conveying piston is manually unlocked.

21. A method according to claim 20, **characterized in that**
the cartridge contents are mixed with a mixing rod which is guided through the conveying piston before the conveying piston is unlocked.

22. A method according to any of Claims 20 or 21, **characterised in that**
the cartridge contains a powder, preferably a cement powder, and a liquid, preferably a monomer liquid, is introduced into the cartridge before the conveying piston is unlocked, in particular before the cartridge contents are mixed.

23. A method according to claim 22, **characterized in that**
an ampoule, preferably a monomer ampoule, is opened to provide the liquid.

24. A method according to any one of claims 20 to 23, **characterised in that**
gas is sucked out of the interior of the cartridge through the conveying piston, wherein preferably the liquid from the ampoule being sucked into the cartridge by the action of a vacuum in the interior of the cartridge.

25. A method according to any of claims 20 to 23, **characterized in that**
the inside of the cartridge and its contents are sterilized by a gas, preferably by ethylene oxide.

## Revendications

1. Cartouche (80) pour extruder un contenu de cartouche, notamment un ciment, de manière particulièrement préférée un ciment médicinal, comprenant au moins une cavité cylindrique délimitée par une paroi de cartouche (72, 73, 92), au moins un piston de transport (1, 21, 41, 61, 81), disposé dans la cavité, mobile le long de l'axe de cylindre dans la cavité, qui est adjacent à la paroi de cartouche (72, 73, 92), et au moins un système de verrouillage (6, 26, 46, 66) avec lequel le piston de transport (1, 21, 41, 61, 81) peut être mis en butée sur la paroi de cartouche (72, 73, 92), où au moins un dispositif de verrouillage (5, 25, 45, 65, 85) est disposé sur le piston de transport (1, 21, 41, 61, 81), qui comprend au moins un des systèmes de verrouillage (6, 26, 46, 66) et qui est accessible à partir de l'extérieur pour un piston de transport (1, 21, 41, 61, 81) verrouillé, **caractérisée en ce que**
chaque dispositif de verrouillage (5, 25, 45, 65, 85) comprend un élément de préhension (7, 27, 47, 67) sous forme d'un prolongement et l'élément de préhension (7, 27, 47, 67), ou les éléments de préhension (7, 27, 47, 67), dépasse, ou dépassent au moins par endroits de la cavité de sorte que le dispositif de verrouillage (6, 25, 45, 65, 85) peut être déverrouillé manuellement.

2. Cartouche (80) selon la revendication 1, **caractérisée en ce que**
le dispositif de verrouillage (5, 25, 45, 65, 85) ou les dispositifs de verrouillage (5, 25, 45, 65, 85) est ou sont solidement reliés avec le piston de transport (1, 21, 41, 61, 81), est ou sont de préférence conçus d'un seul tenant avec le piston de transport (1, 21, 41, 61, 81).

3. Cartouche (80) selon l'une des revendications précédentes, **caractérisée en ce que** deux dispositifs de verrouillage (5, 25, 45, 65, 85), qui sont de préférence disposés l'un en face de l'autre, sont prévus sur le piston de transport (1, 21, 41, 61, 81).

4. Cartouche (80) selon l'une des revendications précédentes, **caractérisée en ce que** le système de verrouillage (6, 26, 46, 66) ou les systèmes de verrouillage (6, 26, 46, 66) est ou sont en prise par-dessus le bord de la paroi de cartouche (72, 73, 92).

5. Cartouche (80) selon l'une des revendications précédentes, **caractérisée en ce que**
le système de verrouillage (6, 26, 46, 66) ou les systèmes de verrouillage (6, 26, 46, 66) se met en prise ou se mettent en prise dans une rainure (74) dans la paroi de cartouche (72, 73, 92), où la rainure (74) est disposée dans une zone de la paroi de cartouche (72, 73, 92) que la partie du piston de transport (1, 21, 41, 61, 81) adjacente à la paroi de cartouche (72, 73, 92) ne recouvre pas lors de l'expulsion de la cartouche (80).

6. Cartouche (80) selon l'une des revendications précédentes, **caractérisée en ce que**
le piston de transport (1, 21, 41, 61, 81) comprend un passage cylindrique.

7. Cartouche selon la revendication 6, **caractérisée en ce**
**qu'**une tige de mélange (29, 49, 69, 89), qui dépasse de la cavité, est disposée dans le passage, avec laquelle on peut mélanger le contenu de la cartouche dans la cavité et qui comprend de préférence un point de rupture souhaitée.

8. Cartouche selon la revendication 7, **caractérisée en ce**
**qu'**une pale de mélange (51, 91) est disposée sur la tige de mélange (29, 49, 69, 89) à l'intérieur de la cartouche (80).

9. Cartouche (80) selon l'une des revendications précédentes, **caractérisée en ce que**
le piston de transport (1, 21, 41, 61, 81) comprend un raccord d'alimentation au vide (8, 28, 48, 68, 88) par lequel le gaz peut être évacué de l'intérieur de la cartouche (80), où de préférence au moins une paroi semi perméable (3, 23, 63, 83), notamment un disque poreux (3, 23, 63, 83), qui est perméable aux gaz et imperméable aux poudres, est disposé dans le passage pour le gaz du raccord d'alimentation au vide (8, 28, 48, 68, 88).

10. Cartouche (80) selon l'une des revendications précédentes, **caractérisée en ce**
**qu'**au moins un dispositif de verrouillage (5, 25, 45, 65, 85), de préférence, que tous les dispositifs de verrouillage (5, 25, 45, 65, 85), peut ou peuvent être déplacés, basculés et/ou pliés en direction de l'axe de cylindre de la cavité, et/ou peut ou peuvent être enlevés par rupture du piston de transport (1, 21, 41, 61, 81), de préférence manuellement.

11. Cartouche (80) selon l'une des revendications précédentes, **caractérisée en ce que**
les systèmes de verrouillage dépassent radialement par rapport à l'axe de cylindre de la cavité au moins par endroits par-dessus le bord de la paroi de cartouche (72, 73, 92) lorsque le piston de transport (1, 21, 41, 61, 81) est en butée.

12. Cartouche (80) selon l'une des revendications précédentes, **caractérisée en ce que**
la cavité est fermée d'un côté par le piston de transport (1, 21, 41, 61, 81) ou est séparée dans une zone à l'intérieur de la cartouche (80) et dans une zone accessible de l'extérieur.

13. Cartouche (80) selon l'une des revendications précédentes, **caractérisée en ce que**
le piston de transport (1, 21, 41, 61, 81) comprend au moins un anneau d'étanchéité (2, 22, 42, 62, 82) qui s'appuie circonférentiellement sur la paroi de cartouche (72, 73, 92).

14. Cartouche (80) selon l'une des revendications précédentes, **caractérisée en ce que**
le piston de transport (1, 21, 41, 61, 81) comprend en dessous du système de verrouillage (6, 26, 46, 66) ou des systèmes de verrouillage (6, 26, 46, 66) respectivement en prolongement des côtés extérieurs du système de verrouillage (6, 26, 46, 66) ou des systèmes de verrouillage (6, 26, 46, 66) au moins une découpe dans l'enveloppe du piston de transport.

15. Cartouche (80) selon l'une des revendications précédentes, **caractérisée en ce que**
la cartouche (80) comprend un orifice de sortie (96), notamment un tuyau de sortie pour l'évacuation du contenu de la cartouche au niveau du côté situé en face du piston de transport (1, 21, 41, 61, 81) dans la position verrouillée.

16. Cartouche (80) selon la revendication 15, **caractérisée en ce**
**qu'**un système de fixation (97), notamment un filetage (97), de préférence un filetage extérieur (97), est agencé dans la zone de l'orifice de sortie (96) de la cartouche (80).

17. Système de cartouche (79) pour préparer un mélange, de préférence, un ciment, de manière particulièrement préférée, un ciment médicinal, comprenant au moins une cartouche (80) selon l'une des revendications précédentes.

18. Système de cartouche (79) selon la revendication 17, **caractérisé en ce que**
le système de cartouche (79) comprend une ampoule (100) avec un contenu liquide, notamment, une ampoule de monomère (100) et que la cartouche (80) contient une poudre, de préférence, une poudre de ciment.

19. Système de cartouche (79) selon la revendication 18, **caractérisé en ce que**
le système de cartouche (79) comprend un mécanisme d'ouverture (104) avec lequel l'ampoule (100) est à ouvrir, et le contenu de l'ampoule (100) peut être mené dans la cartouche (80) par le biais d'une conduite (98), où la conduite (98) est de préférence raccordée à l'orifice de sortie (96) de la cartouche (80).

20. Procédé d'utilisation d'une cartouche selon l'une des revendications 1 à 16, de préférence, moyennant l'utilisation d'un système de cartouche selon l'une des revendications 17 à 19, **caractérisé en ce que**
le piston de transport peut être déverrouillé manuellement.

21. Procédé selon la revendication 20, **caractérisé en ce que**
le contenu de la cartouche est mélangé avec une tige de mélange qui est menée à travers le piston de transport avant que le piston de transport ne soit déverrouillé.

22. Procédé selon l'une des revendications 20 ou 21, **caractérisé en ce que**
la cartouche contient une poudre, de préférence une poudre de ciment et qu'un liquide, de préférence un liquide de monomère, est alimenté dans la cartouche avant que le piston de transport ne soit déverrouillé, notamment avant que le contenu de cartouche ne soit mélangé.

23. Procédé selon la revendication 22, **caractérisé en ce que**
pour la mise à disposition du liquide, une ampoule, de préférence une ampoule de monomère est ouverte.

24. Procédé selon l'une des revendications 20 à 23, **caractérisé en ce que**
du gaz est aspiré de l'intérieur de la cartouche à travers le piston de transport, où de préférence, le liquide est aspiré dans la cartouche hors de l'ampoule par la soumission de l'intérieur de la cartouche à une dépression.

25. Procédé selon l'une des revendications 20 à 23, **caractérisé en ce que**
l'intérieur de la cartouche et le contenu de la cartouche sont stérilisés avec un gaz, de préférence, de l'oxyde d'éthylène.
